# EUROPÄISCHE PATENTSCHRIFT

(11) **EP 0 513 286 B1**
(45) Veröffentlichungstag und Bekanntmachung des Hinweises auf die Patenterteilung: **10.01.1996**
(21) Anmeldenummer: 91920454.5
(22) Anmeldetag: 21.11.1991
(51) Int. Cl.: A61F 2/06

(54) **VORRICHTUNG MIT EINER IN DEN KÖRPER EINES PATIENTEN IMPLANTIERBAREN PROTHESE**
DEVICE WITH A PROSTHESIS IMPLANTABLE IN A PATIENT'S BODY
DISPOSITIF COMPRENANT UNE PROTHESE IMPLANTABLE DANS LE CORPS D'UN PATIENT

(30) Priorität: 26.11.1990 DE 4037507
(43) Veröffentlichungstag der Anmeldung: 19.11.1992
(73) Patentinhaber: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz, Heinrich, Dr.-Ing.
(86) Internationale Anmeldenummer: EP9102194
(87) Internationale Veröffentlichungsnummer: WO9209245

(56) Entgegenhaltungen:
- EP-A- 0 183 372
- EP-A- 0 382 014
- FR-A- 2 333 487
- GB-A- 1 173 811
- US-A- 4 878 906
- US-A- 4 913 141

## Beschreibung

Die Erfindung bezieht sich auf eine Vorrichtung mit einer in den Körper eines Patienten, insbesondere in ein Blutgefäß oder eine andere Körperhöhle, implantierbaren und als Hohlkörper ausgebildeten Prothese, die gegen die Wirkung rückstellender Federkräfte auf einen gegenüber einer - aufgeweiteten - Gebrauchslage reduzierten Querschnitt zusammendrückbar ist sowie nach Wegnahme der die Zusammendrückung vermittelnden Rückhaltekräfte sich selbsttätig auf einen der Gebrauchslage entsprechenden Querschnitt aufweitet.

Vorrichtungen dieser Art sind bekannt und dienen der perkutanen Implantation von insbesondere Gefäßprothesen. Perkutan einführbare und im Lumen sich vergrößernde Prothesen sind entweder mechanisch mittels eines bekannten Ballonkatheters von einem kleinen Radius auf den größeren Radius zum Offenhalten eines Gefäßlumens aufdehnbar oder sie dehnen sich nach vorheriger Zusammendrückung vor der Implantation durch Federkraft, bedingt durch beim Zusammendrücken erzeugte Federvorspannung, von selbst auf.

Es sind bereits verschiedene Systeme bekannt, sich selbst aufdehnende und unter Federkraft stehende Gefäßprothesen in den Körper eines Patienten einzubringen und durch Entfernung der Rückhaltekraft zu implantieren sowie im Gefäß zu verankern.

Die gängigste Methode, die in der EP-A-0 183 372 beschrieben ist, besteht darin, daß eine als schlauchförmiger Hohlkörper ausgebildete Endoprothese auf einen reduzierten Querschnitt zusammengedrückt und dann im zusammengedrückten Zustand mittels eines sogenannten Pushers durch einen zuvor in ein Gefäß eingebrachten Katheter hindurch bis zur lagerichtigen Bestimmung im Gefäß vorgeschoben wird. Bei diesem System besteht jedoch der Nachteil, daß ein erheblicher Kraftaufwand benötigt wird, die Prothese durch den Katheter vorzuschieben, weil der Verschiebung große Reibungskräfte entgegenwirken.

Eine andere Methode (druckschriftlich nicht belegbar) besteht darin, daß eine die Endoprothese bedeckende und diese zusammenhaltende Hülle im Gefäß am Implantationsort zurückgezogen wird. Auch hier besteht der Nachteil, daß große Reibungskräfte überwunden werden müssen. Ferner ist das Schlauchsystem durch die die Prothese bedeckende Hülle recht starr, wodurch das Einführen in ein Gefäß durch Kurven hindurch sehr erschwert ist.

Bei einem weiteren System (US-PS 4 732 152) wird eine gewebte und federnd ausgebildete Prothese im zusammengedrückten Zustand durch eine doppelte Hülle, die am distalen Ende verschlossen ist, zusammengehalten. Diese Hülle wird, wie beim Abstreifen eines Strumpfs vom Fuß eines Trägers, von der zusammengefalteten Prothese zurückgezogen. Zur Verminderung der dabei auftretenden Reibung kann zwischen die beiden Hüllenblätter Flüssigkeit eingefüllt werden. Das wegen der Reduzierung der Reibungswiderstände zunächst elegant erscheinende System ist jedoch äußerst umständlich in der Handhabung und erfordert zwei Personen zur Bedienung.

Darüber hinaus ist aus der US-Patentschrift US-A-4 878 906 eine dünnwandige Prothese bekannt, die mittels eines Ballonkatheters in ein Blutgefäß implantierbar ist. Während der Implantation ist die Prothese von einer gehäkelten oder gestrickten Umhüllung aus Fadenmaterial umschlossen. Das Fadenmaterial ist durch den Katheter aus dem Gefäß herausgeführt und mit einem ebenfalls durch den Katheter geführten Draht verbunden, der die einzelnen Schlaufen der Umhüllung in ihrer die Prothese umschlingenden Lage hält. Dieser Befestigungsdraht verläuft auf der Außenseite der Prothese und ist am distalen Ende des Ballonkatheters lösbar befestigt. Zur Auflösung der Umhüllung wird der Draht und damit die Umhüllung gelöst und zurückgezogen. Anschließend wird mittels des Ballonkatheters die Prothese bestimmungsgemäß aufgeweitet.

Bei dieser Lösung stellt sich das vorgenannte Problem der Reibungskräfte nicht, da die Umhüllung der Prothese nicht unter Überwindung von Rückstellkräften zusammengedrückt wird und demnach beim Zurückziehen der Umhüllung von der Prothese auch nicht entsprechende Reibungskräfte überwunden werden müssen. Die Implantation einer Prothese mittels eines Ballonkatheters gilt jedoch im allgemeinen als umständlich. Dies wird bei dieser Lösung noch dadurch verstärkt, daß zusätzlich zu dem Ballonkatheter auch noch ein Draht und ein Umhüllungsfaden nach der Implantation entfernt werden muß. Darüber hinaus stellt eine etwaige Kompression des Ballons durch die Umhüllung eine Gefährdung des Patienten dar, weil hierdurch der Ballon platzen und damit das Blutgefäß beschädigt werden kann. Ein weiterer Nachteil dieser Lösung besteht darin, daß der Draht außerhalb der Prothese geführt ist und so an der Stelle der Kreuzungspunkte zwischen dem Umhüllungsfaden und dem Befestigungsdraht Unebenheiten und insgesamt eine rauhe Oberfläche gegenüber der Gefäßinnenwand gebildet ist. Außerdem muß der Befestigungsdraht zwischen Prothesen- und Gefäßwand reibend aus dem Blutgefäß entfernt werden.

Demgegenüber soll durch die Erfindung eine besonders einfach und leicht handhabbare Vorrichtung zum Implantieren einer als Hohlkörper ausgebildeten, selbstaufweitenden Prothese geschaffen werden, bei der es sich insbesondere um eine Gefäßprothese handeln kann.

Gelöst ist diese Aufgabe durch die Merkmale des Anspruchs 1.

Bei der Erfindung wird somit die Prothese mittels der diese außenseitig umgebenden Umhüllung in ihrer radial zusammengedrückten Lage gehalten und gelangt erst nach Entfernung dieser Umhüllung, die aufziehbar ausgebildet ist, vermöge der beim Zusammendrücken erzeugten Vorspannkraft in ihre bestimmungsgemäße Aufweitlage.

Die der Erfindung zugrundeliegende Aufgabe wird auch durch die Merkmale des Anspruchs 2 gelöst.

Bei dieser Lösung wird nach lagerichtiger Plazierung der auf einer Sonde oder einem Katheter aufgenommenen Prothese in einem Gefäß zunächst das vom distalen Ende her aufgebrachte Maschenwerk aufgezogen, und zwar beginnend mit der vom distalen Ende entfernten Endmasche und dann allmählich fortschreitend bis zum vollständigen Aufziehen dieses Maschenwerks und dem Zurückziehen des Fadenmaterials. Anschließend daran wird das vom proximalen Ende her aufgebrachte Maschenwerk aufgezogen, und zwar beginnend mit der zum distalen Ende hinweisenden Endmasche und dann fortschreitend in Richtung zum proximalen Ende hin. Es ist ersichtlich, daß bei einem derartigen Aufziehen der Maschenwerke die selbstaufdehnende Prothese sich allmählich fortschreitend von ihrem distalen Ende her in ihre bestimmungsgemäße Gebrauchslage aufweitet.

Bei der Umhüllung kann es sich insbesondere um ein durch Häkeln, Knüpfen, Knoten oder sonstige Arten der Maschenbildung hergestelltes Maschenwerk handeln.

Zweckmäßigerweise kann die mittels der aufziehbaren Umhüllung im radial zusammengedrückten Zustand gehaltene Prothese auf einer Sonde, etwa einem biegsamen Führungsdraht, aufgenommen und auf dieser vorschiebbar sein. Bei einer derartigen Ausgestaltung der Vorrichtung gelingt die Implantation, indem der Führungsdraht in bekannter Weise etwa in ein Gefäß eingebracht und dann die im radial zusammengedrückten Zustand gehaltene Prothese auf dem Führungsdraht vorgeschoben wird, was beispielsweise mittels einer ebenfalls über den Führungsdraht vorgeschobenen und an dem von der Einführseite der Prothese abgewandten Prothesenende angreifenden Hülse vorgeschoben wird.

Eine andere Weiterbildung sieht hingegen vor, daß die mittels der aufziehbaren Umhüllung im radial zusammengedrückten Zustand gehaltene Prothese axialfest auf dem Einführende einer Sonde aufgenommen ist. Bei dieser Sonde kann es sich insbesondere um einen über einen Führungsdraht vorschiebbaren Katheter handeln.

Auch bei der axialfesten Aufnahme der im zusammengedrückten Zustand gehaltenen Prothese auf dem Einführende einer Sonde oder eines Katheters gelingt in einfacher Weise die Implantation, indem die Sonde oder der Katheter mit auf deren Einführende aufgenommener Prothese beispielsweise unter Röntgenkontrolle bis zum Implantationsort vorgeschoben und dann durch Aufziehen der z.B. als umhüllendes Maschenwerk ausgebildeten Umhüllung die Prothese freigegeben und durch deren selbsttätiges Aufdehnen lagerichtig implantiert wird.

Bei der Anordnung der Prothese auf dem Einführende einer Sonde oder eines Katheters hat sich als zweckmäßig erwiesen, wenn die Prothese auf einer die Sonde oder den Katheter umgebenden rutschfesten Unterlage aufgenommen ist, so daß ein unerwünschtes Verrutschen und Abgleiten während des Lösens des das Maschenwerk bildenden Fadenmaterials nicht eintreten kann.

Mit Vorteil kann es sich bei der selbstaufdehnenden Prothese um einen durch Häkeln, Stricken oder sonstige Arten der Maschenbildung hergestellten, aus Metall- und Kunststoff-Fadenmaterial guter Gewebeverträglichkeit bestehenden Schlauch handeln, der gegen die Wirkung von Vorspannkräften radial zusammendrückbar ist und sich nach Wegnahme der Rückhaltekräfte selbsttätig in seine Gebrauchslage aufdehnt und dann in der Aufweitlage verharrt.

Eine besonders zweckmäßige Weiterbildung der Erfindung ist durch die Ausbildung des die Prothese im zusammengedrückten Zustand haltenden schlauchförmigen Maschenwerks in der Weise gekennzeichnet, daß die Maschen nach jeder Umschlingung der Prothese die Richtung ändern und beim Aufziehen aufeinanderfolgender Maschen die letztere bildenden Fadenabschnitte sich abwechselnd rechts- und linksherum von der Prothese lösen.

Der Vorteil dieser Weiterbildung besteht darin, daß die aufeinanderfolgend abwechselnd links- und rechtsherum die Prothese umschlingenden Maschen aufziehbar sind, ohne daß sich dabei das Fadenmaterial um die die Prothese aufnehmende Sonde bzw. einen als solche dienenden Katheter herumschlingt oder eine Verdrillung erfährt, was angesichts der dabei auftretenden Reibung das weitere Zurückziehen des Fadenmaterials erschweren würde.

Als zweckmäßig hat sich bei der vorgenannten Weiterbildung auch erwiesen, wenn die aufziehbaren Schlaufen bzw. Knoten der aufeinanderfolgend die Prothese umschlingenden Maschen einander gegenüber oder aber in einer im wesentlichen axial verlaufenden Reihe hintereinanderliegen.

Eine andere wichtige Weiterbildung der Erfindung sieht vor, daß die Aufziehleine sich von der das Einführende der Prothese umgebenden Masche forterstreckt und mithin die Prothese beim Aufziehen des Maschenwerks von ihrem distalen Ende her allmählich fortschreitend in ihre Aufweitlage gelangt.

Bei dieser Weiterbildung kann das abzuziehende Fadenmaterial beim Aufziehen der Prothese niemals in den Bereich zwischen dem bereits aufgedehnten Prothesenabschnitt und beispielsweise einer Gefäßwand gelangen. Das abzuziehende Fadenmaterial erstreckt sich vielmehr immer nur längs des noch nicht aufgezogenen Maschenwerks und mithin im Bereich der noch in zusammengedrückter Lage gehaltenen Prothese.

Die Enden des das Maschenwerk bildenden Fadenmaterials können mittels aufziehbarer Knoten, etwa in der Form sogenannter Slip-Knoten, festgelegt und dadurch unbeschadet ihrer Aufziehbarkeit sicher gehalten sein. Als besonders einfache Maßnahme zur axialen Fixation der Prothese auf einer Sonde bzw. einem als solche dienenden Katheter hat sich erwiesen, wenn jeweils der Anfang des das Maschenwerk bildenden Fadenmaterials und eine Endmasche in Lochungen der Sonde bzw. des Katheters eingeklemmt, aber mittels der Aufziehleine aus ihren Klemmlagen herausziehbar sind. Der Anfang des Fadenmaterials kann aber auch zwischen der Sonde und einer auf dieser aufgenommenen Manschette eingeklemmt sein.

Besonders sicher gehalten, aber gleichwohl leicht aufziehbar ist das Manschettenwerk, wenn sich von dem Knoten der auf der Aufziehseite des Maschenwerks ersten Masche eine durch eine Lochung hindurchgeführte Schlaufe forterstreckt, deren eines Ende im Bereich des genannten Knotens in die Aufziehleine übergeht. Dadurch ist die genannte Schlaufe mittels der Aufziehleine durch den vorerwähnten Knoten hindurch aufziehbar und danach können nacheinander sämtliche das Maschenwerk bildende Maschen aufgezogen werden.

Im Interesse einer besonders engen Umschließung und dadurch bewirkten Zusammendrückung der Prothese hat sich auch als vorteilhaft erwiesen, wenn zur Bildung des Maschenwerks schrumpffähiges Fadenmaterial verwendet wird. Auch kann das aufziehbare Maschenwerk aus mehreren parallel zueinander verlaufenden Fäden bestehen.

Eine andere wichtige Weiterbildung der Erfindung sieht vor, daß zwischen der Prothese und der diese im radial zusammengedrückten Zustand haltenden Umhüllung wenigstens eine weitere Umhüllung angeordnet ist, welche die Prothese lose umgibt und beim Aufziehen der äußeren Umhüllung ein teilweises Aufdehnen der Prothese zuläßt sowie seinerseits nachfolgend aufziehbar ist.

Bei dieser Weiterbildung handelt es sich mithin darum, daß unmittelbar auf der Prothese eine diese lose und mit gewissem Spiel umgebende Umhüllung aufgenommen ist, bei der es sich um ein Maschenwerk handeln kann, und daß die Prothese und die innere Umhüllung von einer äußeren Umhüllung eng umschlossen sind, welche die Prothese samt der unmittelbar auf dieser aufgenommenen Umhüllung im radial zusammengedrückten Zustand hält. Die Prothese ist folglich gewissermaßen in zwei Lagen umschlossen und vermag nach dem Aufziehen der äußeren Umhüllung sich nur innerhalb der durch die innere Umhüllung gegebenen Grenzen aufzudehnen. Die endgültige Implantation erfolgt dann durch Aufziehen der inneren Umhüllung, also in Stufen.

Selbstverständlich können auch mehrere einander mit gewissem Spiel umschließende Maschenwerke vorgesehen sein, die ein Aufdehnen der Prothese in mehreren aufeinanderfolgenden Stufen ermöglichen.

Im Rahmen der Erfindung können auch die Zwischenräume zwischen den Maschen eines die Prothese umschließenden und im zusammengedrückten Zustand haltenden Maschenwerks mit Gelatine oder einer ähnlichen Substanz, die sich im Körper eines Patienten auflöst, ausgefüllt und geglättet sein. Dadurch wird das Einführen einer derartigen Vorrichtung erleichtert.

Gemäß einer nochmaligen Weiterbildung kann auch wenigstens ein Ende der Prothese im zusammengedrückten Zustand von einer Manschette übergriffen sein, das infolge der beim Aufdehnen eintretenden axialen Verkürzung der Prothese aus der durch die Manschette vermittelten Halterung gelangt. Eine derartige Manschette kann mit der zur Prothese hinweisenden Offenseite fest auf der Sonde bzw. einem Katheter beispielsweise auf der zum distalen Ende hinweisenden Seite angeordnet sein. Dadurch ist ein das Einführen erleichternder glatter Übergang an dem in Einführrichtung vorderen Prothesenende verwirklicht.

Im Interesse einer verbesserten Fixation der Prothese auf einer Sonde oder einem als solche dienenden Katheter kann sich das von der Einführseite abgewandte Ende der Prothese an einer radial vorstehenden Stufe oder Schulter bzw. an einer auf der Sonde bzw. dem Katheter aufgenommenen Manschette abstützen.

Eine abermalige Weiterbildung der Erfindung sieht vor, daß bei Verwendung eines Katheters als Sonde die Aufziehleine durch eine die Katheterwandung in der Nähe eines Endes der Prothese durchbrechende Lochung in das Katheter-Lumen eingeführt ist, sich durch letzteres hindurch erstreckt und bis über das Ende des Katheters reicht.

Es kann aber auch ein doppellumiger Katheter als Sonde dienen, wobei ein Lumen zum Vorschieben des Katheters über einen Führungsdraht dient und durch das andere Lumen die Aufziehleine hindurchgeführt ist.

Bei der Verwendung eines ein- oder doppellumigen Katheters als Sonde mit durch das Katheterlumen hindurchgeführter Aufziehleine ist sichergestellt, daß von der Aufziehleine bzw. beim Aufziehen des Maschenwerks von dem dann durch das Katheterlumen zurückzuziehenden Fadenmaterial die Wandungen von Gefäßen oder sonstigen Körperhöhlen, in die eine Prothese implantiert werden soll, nicht geschädigt werden können.

Als vorteilhaft hat sich auch erwiesen, wenn die Aufziehleine und/oder das Fadenmaterial des Maschenwerks mit einem reibungsmindernden Gleitmittel ausgerüstet ist.

Auch kann wenigstens die Aufziehleine als Metallfaden ausgebildet oder mit einer Beimischung aus Metall ausgerüstet sein, so daß eine gute Darstellbarkeit im Röntgenbild gewährleistet ist.

Schließlich kann auch, gemäß einer abermaligen Weiterbildung, die mittels der aufziehbaren Umhüllung in radial zusammengedrückter Lage gehaltene Prothese sich - im aufgeweiteten Zustand nach Entfernung der Umhüllung - zu ihrem proximalen Ende hin trompetenartig erweitern. Dieser Prothesenausbildung kommt bei Implantationen im Bereich von Gefäßabzweigungen Bedeutung zu, weil dann immer die Gefahr des Abgleitens der Prothese in das abzweigende Gefäß besteht. Angesichts der trompetenartigen Erweiterung am proximalen Ende ist jedenfalls ein derartiges Abgleiten bei der Implantation wirksam unterbunden, wenn die die Prothese umschließende Umhüllung vom proximalen Ende her aufgezogen wird.

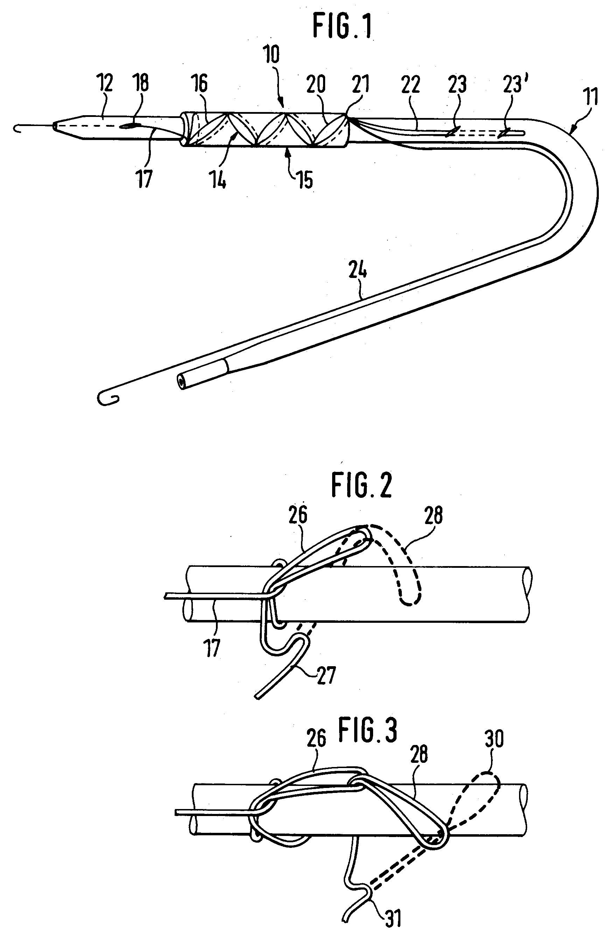

Eine Ausführungsform der erfindungsgemäßen Vorrichtung soll nachstehend anhand der beigefügten Zeichnung erläutert werden. In schematischen Ansichten zeigen:
- Fig. 1: einen Katheter mit einer auf dessen distalem Ende aufgenommenen und durch eine Umhäkelung in Form eines aufziehbaren schlauchartigen Maschenwerks unter radialer Vorspannung in zusammengedrückter Lage gehaltenen Gefäßprothese,
- Fig. 2: ein Schaubild zur Verdeutlichung der Bildung einer Anfangsmasche der Prothesen-Umhäkelung mit einer rechtsseitig um die Gefäßprothese herumgeführten Schlaufe,
- Fig. 3: ein Schaubild wie in Fig. 2 zur Verdeutlichung der Bildung einer sich an die Anfangsmasche anschließenden Häkelmasche mit linksseitiger Umschlingung der Gefäßprothese,
- Fig. 4: in einer Ansicht ähnlich wie in Fig. 1 eine Vorrichtungsausbildung, bei der die auf dem Katheter aufgenommene Gefäßprothese mittels je einer vom distalen und proximalen Ende her aufgebrachten aufziehbaren Umhäkelung unter radialer Vorspannung in ihrer zusammengedrückten Lage gehalten wird,
- Fig. 5: die Vorrichtung gemäß Fig. 4 lediglich mit der vom distalen Ende her aufgebrachten Umhäkelung,
- Fig. 6: die Vorrichtung gemäß Fig. 4 mit der vom proximalen Ende her aufgebrachten Umhäkelung allein bei Weglassung der in Fig. 5 veranschaulichten Umhäkelung,
Bei der in Fig. 1 veranschaulichten Vorrichtung 10 dient als Sonde ein langgestreckter Katheter 11 mit einem durchgehenden Lumen, mittels dessen der Katheter über einen in bekannter Weise in ein Gefäß eingebrachten Führungsdraht vorgeschoben werden kann. In der Nähe seines distalen Endes 12 befindet sich auf dem Katheter 11 eine mittels einer Umhäkelung 14 unter radialer Vorspannung in einer zusammengedrückten Lage gehaltene Prothese 15, die nach Wegfall der von der Umhäkelung vermittelten Rückhaltekraft unter Selbstaufdehnung in ihre bestimmungsgemäße Aufweitlage geht. Beispielsweise kann es sich bei der Prothese um ein schlauchförmiges Gestricke handeln, das gegen die Wirkung einer rückstellenden Federkraft radial in eine den Katheter in der Nähe seines distalen Endes eng umschließende Lage zusammendrückbar ist.

Umschlossen ist die Prothese 15 von einer aus einem durchgehenden Faden gebildeten Umhäkelung 14, bei der die aufeinanderfolgenden Maschen die Prothese abwechselnd auf der einen oder anderen Seite, also abwechselnd rechts- oder linksseitig umschlingen. Der vor der dem distalen Ende 12 des Katheters 11 zugeordneten ersten Masche 16 liegende Anfangsabschnitt 17 des Fadenmaterials ist durch einen Schlitz 18 in der Katheterwand hindurchgezogen, in dem genannten Schlitz eingeklemmt und erstreckt sich dann durch das Katheterlumen hindurch und bis über das distale Ende des Katheters hinaus. Durch einen die vom distalen Ende entfernte Endmasche 20 abschließenden Knoten 21 ist eine aufziehbare Schlaufe 22 hindurchgezogen, die durch zwei beanstandete Einschnitte 23, 23' in der Katheterwand hindurchgezogen und somit ebenfalls infolge Klemmung axial festgelegt ist.

Das durch den Knoten 21 der genannten Endmasche 20 hindurchgeführte freie Fadenende bildet eine sich längs des Katheters 11 erstreckende Aufziehleine 24, mittels der durch den genannten Endknoten hindurch zunächst die am Katheter durch Klemmung gehaltene Schlaufe 22 und dann nach und nach die sich um die Prothese herumerstreckenden und diese in ihrer zusammengedrückten Lage haltenden Maschen der Umhäkelung aufgezogen werden können. Da die Maschen abwechselnd die Prothese 15 rechts- und linksseitig umschlingen, löst sich beim Aufziehen der Maschen jeweils abwechselnd der Faden auf der rechten und linken Seite des Katheters vom zugeordneten Maschenknoten und nach dem Lösen der dem distalen Ende zugewandten Masche ist der Anfangsabschnitt 17 des Fadenmaterials aus seiner Klemmlage im Schlitz 18 am distalen Ende 12 des Katheters 11 herausziehbar.

In vergrößerter Darstellung veranschaulichen die Fig. 2 und 3 die Maschenbildung mit abwechselnd vorder- und rückseitiger Umschlingung des Katheters 15, der in diesen Figuren zur Einfachheit als starres schlauchförmiges Gebilde dargestellt ist. Nach dem Festlegen des Anfangsabschnittes 17 des Fadenmaterials in der aus Fig. 1 ersichtlichen Weise durch Klemmung in dem Schlitz 18 wird der Faden um den Katheter herumgeschlungen, dann unter dem Faden eine Schlaufe 26 hindurchgezogen und danach von dem freien Fadenmaterial 27 eine Masche auf der Rückseite des Katheters um diesen herum und durch die Schlaufe 26 hindurchgezogen, deren durch die erstgenannte Schlaufe 26 gezogener Abschnitt wiederum eine Schlaufe 28 für die Bildung der nächstfolgenden Masche bildet. Fig. 2 zeigt in vollen Linien das freie Fadenmaterial 27 vor dem Durchziehen durch die Schlaufe 26 und in gestrichelter Linie nach dem Durchziehen durch diese Schlaufe und der Bildung der Schlaufe 28 für die nächste Masche.

Zur nächsten Maschenbildung, die in Fig. 3 veranschaulicht ist, wird in der in gestrichelten Linien angedeuteten Weise unter Bildung einer weiteren Schlaufe 30 das freie Fadenmaterial aus der mit 31 bezeichneten Lage vor dem Katheter durch die zuvor gebildete Schlaufe 28 hindurchgezogen und dann diese Art der Schlaufen- und Maschenbildung mit abwechselnd hinter und vor dem Katheter durch die jeweilige Schlaufe gezogenem Fadenmaterial fortgesetzt, bis die auf dem Katheter aufgenommene Prothese auf ihrer gesamten Länge umhäkelt ist.

Die zur Bildung der Endmasche 20 durch die dieser zugeordnete Schlaufe bzw. einen durch Zusammenziehen dieser Schlaufe gebildeten Knoten 21 hindurchgezogene Schlaufe 22 wird dann in der in Fig. 1 schematisch dargestellten Weise durch die zwei axial beabstandeten Schlitzungen 23, 23' in der Wandung des Katheters hindurchgezogen und durch Einklemmung festgelegt. Das verbleibende Fadenmaterial bildet dann die Aufziehleine 24, die sich von der Schlaufe der Endmasche 20 forterstreckt und ein Aufziehen der Umhäkelung ermöglicht, wobei das Fadenmaterial der jeweils aufgezogenen Maschen sich abwechselnd auf der einen und anderen Seite der Prothese 15 löst und dadurch die Prothese unter der beim Umhäkeln infolge der dabei erfolgten radialen Zusammendrückung aufgeprägten Vorspannkraft zur Aufdehnung freigibt.

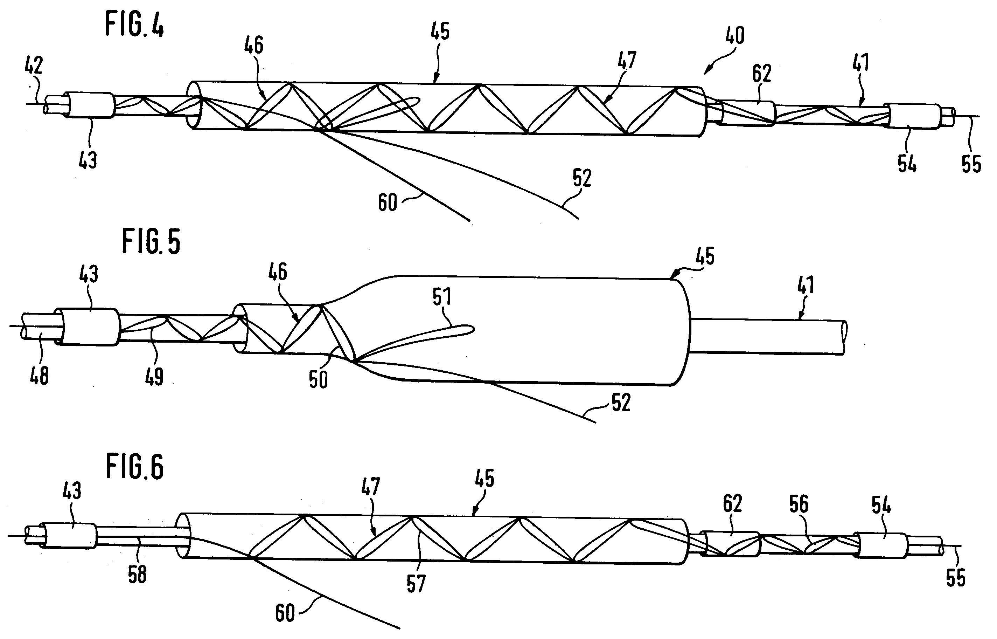

Auch bei der in Fig. 4 gezeigten Ausführungsform 40 ist auf einem langgestreckten Katheter 41 in der Nähe des distalen Katheterendes 42 eine Prothese 45 aufgenommen und unter radialer Vorspannung in ihrer zusammengedrückten Lage gehalten. Diesem Zwecke dienen in den Fig. 5 und 6 jeweils für sich gezeigte Umhäkelungen 46, 47. Der Katheter 41 ist, ebenso wie der Katheter 11 der Ausführungsform nach Fig. 1, über einen zuvor in ein Gefäß eingebrachten Führungsdraht im Gefäß vorschiebbar und dadurch die auf dem Katheter aufgenommene Prothese 45 vor ihrer Implantation durch Aufziehen der Umhäkelungen lagerichtig im Gefäß implantierbar.

Die beiden die Prothese 45 in der in Fig. 4 veranschaulichten zusammengedrückten Lage haltenden Umhäkelungen werden nacheinander aufgebracht, und zwar zunächst die Umhäkelung 46 vom distalen Ende aus. Die andere Umhäkelung 47 wird vom proximalen Ende aus aufgebracht und überdeckt dann das Ende der erstgenannten Umhäkelung 46.

In Fig. 5 ist gezeigt, daß der Katheter 41 am distalen Ende mit einer Silikonmanschette 43 ausgerüstet ist, die der Fixierung des Anfangsabschnittes 48 des für die Ausbildung der ersten Umhäkelung notwendigen Fadens dient. Aus diesem Grunde ist der Anfangsabschnitt 48 dieses Fadens Unter der Silikonmanschette 43 hindurchgezogen. Danach sind die ersten Maschen 49 in der oben in Verbindung mit den Fig. 1 bis 3 erläuterten Weise auf dem Katheter 41 gehäkelt, die für die erste Umhäkelung einen festen Sitz auf dem Katheter vermitteln. Die anschließenden Maschen 50 übergreifen das zum distalen Katheterende hinweisende Ende der Prothese 41 und drücken dieses unter radialer Vorspannung bei gleichzeitiger axialer Fixation der Prothese auf dem Katheter zusammen, wie dies in Fig. 5 veranschaulicht ist. Eine letzte Masche 51 dieser Umhäkelung 46 wird dann außenseitig auf der Prothese 45 abgelegt und von dieser Masche erstreckt sich als Leine zum Aufziehen der Maschen der genannten Umhäkelung der Faden 52 fort.

Fig. 6 veranschaulicht die Aufbringung der zweiten Umhäkelung 47 vom proximalen Ende her. Der Anfang 55 des Fadenmaterials dieser Umhäkelung wird wieder mittels einer am proximalen Ende auf dem Katheter 41 aufgezogenen Silikonmanschette 54 festgelegt, indem der Fadenanfang unter dieser Manschette hindurchgezogen wird. Anschließend werden in Richtung auf das distale Ende hin auf dem Katheter einige Maschen 56 gehäkelt und anschließend weitere Maschen 57 unter Umschlingung der Prothese 41 bei deren gleichzeitiger radialer Zusammendrückung bis über die vom distalen Ende her abgewandten Maschen 50, 51 der ersten Umhäkelung 46 hinaus, die dadurch festgelegt werden. Eine letzte Masche 58 der vom proximalen Ende her aufgebrachten Umhäkelung 47 wird dann unter der am distalen Ende des Katheters auf diesen aufgeschobenen Silikonmanschette 43 hindurchgezogen und dadurch festgelegt. Im übrigen erstreckt sich von der dem distalen Ende zugewandten Endmasche der vom proximalen Ende her aufgebrachten Umhäkelung 47 als Leine zum Aufziehen der Maschen dieser Umhäkelung der Faden 60 fort.

Die Prothese 45 der in den Fig. 4 bis 6 veranschaulichten Ausführungsform ist, ebenso wie bei der Ausführungsform nach den Fig. 1 bis 3, unter radialer Vorspannung in zusammengedrückter Lage auf dem Katheter 41 gehalten und dehnt sich nach dem Entfernen der Umhäkelungen 46, 47 selbsttätig in ihre Aufweitlage auf. Nach dem Einführen der auf dem Katheter aufgenommenen Prothese in ein Gefäß und lagerichtiger Plazierung dort erfolgt die Implantation in der Weise, daß zunächst die vom distalen Ende her aufgebrachte Umhäkelung 46 entfernt wird. Dies geschieht durch Aufziehen der Maschen dieser Umhäkelung mittels der Aufziehleine 52, indem zunächst die unter dem distalen Ende der von der proximalen Seite her aufgebrachten Umhäkelung liegende Masche 51 und nach und nach dann die sich zum distalen Ende hin anschließenden Maschen 50 und 49 aufgezogen werden, bis sich zuletzt die der Silikonmanschette 43 benachbarte erste Masche löst und der Fadenanfang 48 unter der Silikonmaschette herausgezogen wird.

Da durch Aufziehen der vom distalen Ende her aufgebrachten Umhäkelung 46 das zum distalen Katheterende hinweisende Ende der Prothese 45 freigegeben wird, dehnt sich dieses Prothesenende infolge der der Prothese eigenen Vorspannkräfte radial auf, während der übrige Teil der Prothese noch durch die vom proximalen Ende her aufgebrachte Umhäkelung 47 in zusammengedrückter Lage gehalten wird. Axial fixiert ist in dieser Lage die teils aufgeweitete Prothese 45 einerseits durch die Haftwirkung zwischen Katheter und Prothese und andererseits durch eine am proximalen Ende der Prothese 45 auf dem Katheter 41 aufgenommene Silikonmanschette 62, an der sich die Prothese axial abstützt.

Nach dem Aufziehen der ersten Umhäkelung 46 wird auch die vom proximalen Ende her aufgebrachte Umhäkelung 47 aufgezogen, und zwar mittels der sich von deren Endmasche 58 auf der zum distalen Ende hinweisenden Seite forterstreckenden Aufziehleine 60. Es ist ersichtlich, daß durch Ziehen an der Aufziehleine zunächst die unter der Silikonmanschette 43 am distalen Ende festgelegte Schlaufe 58 aufgezogen wird und dann das Aufziehen der Maschen 57 und 56 von der dem distalen Ende zugewandten Seite fortschreitend in Richtung zum proximalen Ende stattfindet, wobei die Prothese 45 sich radial aufweitet und an den Wandungen eines mit der Prothese auszurüstenden Gefäßes anlegt. Am Schluß des Aufziehvorganges wird das unter der Silikonmanschette 54 am proximalen Ende festgelegte Fadenende 55 herausgezogen. Die Prothese 45 ist dann vom Katheter 41 gelöst und letzterer kann in einfacher Weise aus dem Gefäß zurückgezogen werden.

## Patentansprüche

1. Vorrichtung mit einer in den Körper eines Patienten, insbesondere in ein Blutgefäß oder eine andere Körperhöhle, implantierbaren und als Hohlkörper ausgebildeten Prothese, die gegen die Wirkung rückstellender Federkräfte auf einen gegenüber einer - aufgeweiteten Gebrauchslage reduzierten Querschnitt zusammendrückbar ist sowie nach Wegnahme der die Zusammendrückung vermittelnden Rückhaltekräfte sich selbsttätig auf einen Querschnitt aufweitet,
dadurch gekennzeichnet,
daß die selbstaufweitende Prothese (15) von einer aufziehbaren Umhüllung (14), die aus einem durchlaufenden Faden besteht, umschlossen und auf einen reduzierten Querschnitt zusammengedrückt ist und daß der Faden sich von der Umhüllung als Aufziehleine (24), an der die Umhüllung auf- und zurückziehbar ist, forterstreckt.

2. Vorrichtung mit einer in den Körper eines Patienten insbesondere in ein Blutgefäß oder eine andere Körperhöhle, implantierbaren und als Hohlkörper ausgebildeten Prothese, die gegen die Wirkung rückstellender Federkräfte auf einen gegenüber einer - aufgeweiteten Gebrauchslage reduzierten Querschnitt zusammendrückbar ist sowie nach Wegnahme der die Zusammendrückung vermittelnden Rückhaltekräfte sich selbsttätig auf einen Querschnitt aufweitet,
dadurch gekennzeichnet,
daß die selbstaufweitende Prothese (45) mittels eines aus einem durchlaufenden Faden bestehenden von einem distalen Ende (42) einer Sonde bzw. eines Katheters (41) her aufgebrachten und bis über ein Einführende der Prothese (45) reichenden Maschenwerks (46) und mittels eines aus einem zweiten durchlaufenden Faden bestehenden dazu gegenläufig von dem anderen proximalen Ende der Sonde bzw. des Katheters (41) her aufgebrachten sowie über die Endmaschen (51) des erstgenannten Maschenwerks (46) reichenden zweiten Maschenwerks (47) in seiner radial zusammengedrückten Lage gehalten wird und daß die beiden Maschenwerke (46, 47) jeweils von ihren schlaufenförmigen Endmaschen (51, 58) her mittels den von den Endmaschen sich jeweils als Aufziehleine (52, 60) forterstreckenden genannten Fäden gegenläufig aufziehbar sind.

3. Vorrichtung nach Anspruch 1 oder 2, dadurch gekennzeichnet, daß es sich bei der die Prothese (15, 45) in ihrer radial zusammengedrückten Lage haltenden Umhüllung um ein aufziehbares Maschenwerk (14, 46, 47) handelt.

4. Vorrichtung nach Anspruch 1, 2 oder 3, dadurch gekennzeichnet, daß die mittels der aufziehbaren Umhüllung (14, 46, 47, 74) im radial zusammengedrückten Zustand gehaltene Prothese (15, 45) auf einer Sonde (11, 41), etwa einem biegsamen Führungsdraht, aufgenommen und auf dieser vorschiebbar ist.

5. Vorrichtung nach Anspruch 1, 2 oder 3, dadurch gekennzeichnet, daß die mittels der aufziehbaren Umhüllung (14, 46, 47) im radial zusammengedrückten Zustand gehaltene Prothese (15, 45) axialfest auf dem Einführende der Sonde (11, 41) aufgenommen ist.

6. Vorrichtung nach Anspruch 5, dadurch gekennzeichnet, daß es sich bei der die Prothese (15, 45) auf ihrem Einführende (12) aufnehmenden Sonde um einen über einen Führungsdraht vorschiebbaren Katheter (11, 41) handelt.

7. Vorrichtung nach Anspruch 5 oder 6, dadurch gekennzeichnet, daß die Prothese (15, 45) unter Zwischenlage einer rutschfesten Unterlage auf der Sonde aufgenommen ist.

8. Vorrichtung nach einem der Ansprüche 1 bis 6, dadurch gekennzeichnet, daß es sich bei der Prothese (15, 45) um einen durch Häkeln, Stricken oder sonstige Arten der Maschenbildung hergestellten, aus Metall- oder Kunststoff-Fadenmaterial guter Gewebeverträglichkeit bestehenden Schlauch handelt, der gegen Vorspannkräfte radial zusammendrückbar ist und sich nach Wegnahme der Rückhaltekräfte selbsttätig in seine Gebrauchslage aufdehnt und dann in der Aufweitlage verharrt.

9. Vorrichtung nach einem der Ansprüche 1 bis 8, gekennzeichnet durch die schlauchförmige Ausbildung des die Prothese (15, 45) im zusammengedrückten Zustand haltenden Maschenwerks (14, 46, 47) in der Weise, daß die Maschen nach jeder Umschlingung der Prothese die Richtung ändern und beim Aufziehen aufeinanderfolgender Maschen die letztere bildenden Fadenabschnitte sich abwechselnd rechts- und linksherum von der Prothese lösen.

10. Vorrichtung nach Anspruch 9, dadurch gekennzeichnet, daß es sich bei dem die Prothese (15, 45) umhüllenden Maschenwerk (14, 46, 47) um einen durch Häkeln, Knüpfen, Knoten oder sonstige Arten der Maschenbildung hergestellten aufziehbaren Schlauch handelt.

11. Vorrichtung nach Anspruch 9 oder 10, dadurch gekennzeichnet, daß die aufziehbaren Knoten der aufeinanderfolgend die Prothese (15, 45) umschlingenden Maschen des umhüllenden Maschenwerks (14, 46, 47) jeweils einander gegenüberliegen oder in einer im wesentlichen axial verlaufenden Reihe hintereinanderliegend angeordnet sind.

12. Vorrichtung nach einem der Ansprüche 1 bis 11, dadurch gekennzeichnet, daß die Aufziehleine (24, 52) sich von den das Einführende der Prothese (15, 45) umgebenden Maschen forterstreckt und mithin die Prothese beim Aufziehen des Maschenwerks von ihrem Einführende her allmählich fortschreitend in ihre Aufweitlage gelangt.

13. Vorrichtung nach einem der Ansprüche 1 bis 12, dadurch gekennzeichnet, daß die Enden des das Maschenwerk (14, 46, 47) bildenden Fadenmaterials mittels aufziehbarer Knoten, etwa in der Form sogenannter Slip-Knoten, festgelegt sind.

14. Vorrichtung nach einem der Ansprüche 3 bis 13, dadurch gekennzeichnet, daß jeweils der Anfang des das Maschenwerk (14, 45, 46) bildenden Fadenmaterials und eine Endmasche an der Sonde bzw. dem als solche dienenden Katheter (11, 41) festgelegt und mittels der Aufziehleine (24, 52, 60) aus ihren Festlegungen herausziehbar sind.

15. Vorrichtung nach Anspruch 14, dadurch gekennzeichnet, daß der Anfang (17, 48, 55) des das Maschenwerk (14, 46, 47) bildenden Fadenmaterials und dessen Endmasche (22, 51, 58) in Lochungen (18, 23, 23') der Sonde bzw. dem als solche dienenden Katheter eingeklemmt sind.

16. Vorrichtung nach Anspruch 15, dadurch gekennzeichnet, daß der Anfang (48, 55) des das Maschenwerk (46, 47) bildenden Fadenmaterials zwischen der Sonde bzw. dem Katheter und einer auf diese bzw. diesen aufgeschobenen elastischen Manschette (43, 54) eingeklemmt ist.

17. Vorrichtung nach einem der Ansprüche 14 bis 16, dadurch gekennzeichnet, daß sich von dem Knoten der auf der Aufziehseite des Maschenwerks (14, 46, 47) ersten Masche eine an der Sonde bzw. dem Katheter festgelegte Schlaufe (22, 51, 58) forterstreckt, deren eines Ende im Bereich des genannten Knotens in die Aufziehleine (24, 52, 60) übergeht und die mittels letzterer durch den vorerwähnten Knoten hindurch aufziehbar ist.

18. Vorrichtung nach einem der Ansprüche 1 bis 17, gekennzeichnet durch Verwendung schrumpffähigen Fadenmaterials zur Bildung des die Prothese (15, 45) im zusammengedrückten Zustand auf der Sonde bzw. einem als solche dienenden Katheter (11, 41) umschließenden Umhüllung (14, 46, 47).

19. Vorrichtung nach einem der Ansprüche 2 bis 18, dadurch gekennzeichnet, daß die aufziehbare Umhüllung (14, 46, 47) aus mehreren parallel zueinander verlaufenden Fäden besteht.

20. Vorrichtung nach einem der Ansprüche 1 bis 19, dadurch gekennzeichnet, daß zwischen der Prothese (15, 45) und der diese im radial zusammengedrückten Zustand haltenden Umhüllung (14, 46, 47) wenigstens eine weitere Umhüllung angeordnet ist, welche die Prothese lose umgibt und beim Aufziehen der äußeren Umhüllung ein teilweises Aufdehnen der Prothese zuläßt sowie seinerseits nachfolgend aufziehbar ist.

21. Vorrichtung nach einem der Ansprüche 1 bis 19, dadurch gekennzeichnet, daß die Zwischenräume zwischen den Maschen des die Prothese (15, 45) umschließenden und im zusammengedrückten Zustand haltenden Maschenwerks (14, 46, 47) mit Gelatine oder einer ähnlichen Substanz, die sich im Körper eines Patienten auflöst, ausgefüllt und geglättet sind.

22. Vorrichtung nach einem der Ansprüche 6 bis 21, dadurch gekennzeichnet, daß wenigstens ein Ende der Prothese (15, 45) im zusammengedrückten Zustand von einer Manschette der Sonde bzw. des Katheters übergriffen ist und infolge der beim Aufdehnen eintretenden axialen Verkürzung aus der durch die Manschette vermittelten Halterung gelangt.

23. Vorrichtung nach einem der Ansprüche 5 bis 22, dadurch gekennzeichnet, daß sich das von der Einführseite abgewandte Ende der Prothese (15, 45) an einer radial vorstehenden Stufe oder Schulter der Sonde bzw. an einer auf letzterer aufgenommenen Manschette (62) abstützt.

24. Vorrichtung nach einem der Ansprüche 6 bis 23, dadurch gekennzeichnet, daß bei Verwendung eines Katheters (11, 41) als Sonde die Aufziehleine (24, 52, 60) durch eine die Katheterwandung in der Nähe eines Endes der Prothese durchbrechende Lochung in das Katheter-Lumen eingeführt ist, sich durch letzteres hindurcherstreckt und bis über das Ende des Katheters reicht.

25. Vorrichtung nach einem der Ansprüche 6 bis 24, gekennzeichnet durch einen doppellumigen Katheter als die Prothese (15, 45) aufnehmende Sonde, wobei ein Lumen zum Vorschieben des Katheters über einen Führungsdraht dient und durch das andere Lumen die Aufziehleine für das die Prothese umgebende Maschenwerk hindurchgeführt ist.

26. Vorrichtung nach einem der Ansprüche 1 bis 25, gekennzeichnet durch die Ausrüstung des die Aufziehleine (24, 52, 60) und/oder die Umhüllung (14, 46, 47) bildenden Fadenmaterials mit einem reibungsmindernden Gleitmittel.

27. Vorrichtung nach einem der Ansprüche 1 bis 26, dadurch gekennzeichnet, daß wenigstens die Aufziehleine (24, 52, 60) als Metallfaden ausgebildet bzw. mit einer Beimischung aus im Röntgenbild darstellbarem Metall ausgerüstet ist.

28. Vorrichtung nach einem der Ansprüche 1 bis 27, dadurch gekennzeichnet, daß die mittels der aufziehbaren Umhüllung (14, 46, 47) in radial zusammengedrückter Lage gehaltene Prothese (15, 45) sich - im aufgeweiteten Zustand nach Entfernung der Umhüllung - zu ihrem proximalen Ende hin trompetenartig erweitert.

## Claims

1. Device with a prothesis, which is formed as hollow body and implantable into the body of a patient, in particular into a blood vessel or another body cavity and which is compressible to a cross-section, which is reduced by comparison with an expanded condition of use, against the effect of restoring spring forces as well as expands automatically to a cross-section after removal of the restraining forces providing the compression, characterised thereby, that the self-expanding prothesis (15) is surrounded by a sheathing (14), which is drawable onto the prothesis, consists of a continuous thread and is compressed to a reduced cross-section, and that the thread extends forth from the sheath as pull line (24), by which the sheathing can be drawn on and back.

2. Device with a prothesis, which is formed as hollow body and implantable into the body of a patient, in particular into a blood vessel or another body cavity and which is compressible to a cross-section, which is reduced by comparison with an expanded condition of use, against the effect of restoring spring forces as well as expands automatically to a cross-section after removal of the restraining forces providing the compression, characterised thereby, that the self-expanding prothesis (45) is held in its radially compressed condition by means of a mesh (46), which consists of a continuous thread, is applied from a distal end (42) of a probe or a catheter (41) and reaches over an entering end of the prothesis (45), and by means of a second mesh (47), which consists of a second continuous thread and is applied in opposite direction from the other proximal end of the probe or catheter (41) as well as reaches over the end stitches (51) of the first-mentioned mesh (46) and that both the meshes (46, 47) are each drawable on from their loop-shaped end stitches (51, 58) by means of the mentioned threads extending forth from the end stitches each time as pull line (52, 60).

3. Device according to claim 1 or 2, characterised thereby, that a mesh (14, 46, 47), which can be drawn on, is concerned in the case of the sheathing holding the prothesis (15, 45) in its radially compressed condition.

4. Device according to claim 1, 2 or 3, characterised thereby, that the prothesis (15, 45), which is held in the radially compressed condition by means of the sheathing (14, 46, 47, 74), which can be drawn on, is received on a probe (11, 41), possibly a flexible guide wire, and advanceable on this.

5. Device according to claim 1, 2 or 3, characterised thereby, that the prothesis (15, 45), which is held in the radially compressed condition by means of the sheathing (14, 46, 47), which can be drawn on, is received axially fast on the entering end of the probe (11, 41).

6. Device according to claim 5, characterised thereby, that a catheter (11, 41), which is advanceable by way of a guide wire, is concerned in the case of the probe receiving the prothesis (15, 45) on its entering end (12).

7. Device according to claim 5 or 6, characterised thereby, that the prothesis (15, 45) is received on the probe with the interposition of a slip-resistant underlay.

8. Device according to one of the claims 1 to 6, characterised thereby, that a hose, which consists of metal or synthetic material threads of good tissue compatibility, is produced by crocheting, knitting or other kinds of the mesh formation, is radially compressible against biassing forces, expands automatically into its condition of use after removal of the restraining forces and then remains in the expanded condition, is concerned in the case of the prothesis (15, 45).

9. Device according to one of the claims 1 to 8, characterised by the hose-shaped formation of the mesh (14, 46, 47), which holds the prothesis (15, 45) in the compressed state, in the manner that the stitches change in direction after each looping of the prothesis and, during the pulling-up of successive stitches, the thread portions forming the latter detach from the prothesis alternately in clockwise and anticlockwise sense.

10. Device according to claim 9, characterised thereby, that a hose, which can be drawn on and is made by crocheting, meshing or knotting or other kinds of the mesh formation, is concerned in the case of the mesh (14, 46, 47) sheathing the prothesis (15, 45).

11. Device according to claim 9 or 10, characterised thereby, that the knots, which can be drawn tight, of the stitches, which successively loop around the prothesis (15, 45), of the sheathing mesh (14, 46, 47) each time lie one opposite the other or are arranged to lie one behind the other in a substantially axially extending row.

12. Device according to one of the claims 1 to 11, characterised thereby, that the pull line (24, 52) extends forth from the stitches surrounding the entering end of the prothesis (15, 45) and the prothesis thereby gets progressively into its expanded condition gradually during the drawing-on of the mesh from its entering end.

13. Device according to one of the claims 1 to 12, characterised thereby, that the ends of the thread material forming the mesh (14, 46, 47) are fixed by means of tightenable knots, possibly in the form of so-called slip knots.

14. Device according to one of the claims 3 to 13, characterised thereby, that the start of the thread material forming the mesh (14, 46, 47) each time and an end stitch are fixed at the probe or the catheter (11, 41) serving as such and drawable out of their fixings by means of the pull line (24, 52, 60).

15. Device according to claim 14, characterised thereby, that the start (16, 48, 55) of the thread material forming the mesh (14, 46, 47) and its end stitch (22, 51, 58) are wedged into holes (18, 23, 23') of the probe or the catheter serving as such.

16. Device according to claim 15, characterised thereby, that the start (48, 55) of the thread material forming the mesh (14, 46, 47) is clamped in between the probe or the catheter and an elastic sleeve (43, 54) pushed onto this.

17. Device according to one of the claims 14 to 16, characterised thereby, that a loop (22, 51, 58), which is fixed at the probe or the catheter and the one end of which passes over into the pull line (24, 52, 60) in the region of the mentioned knot and which is tightenable through the aforementioned knot by means of the pull line, extends forth from the knot of the first stitch on the tightening side of the mesh (14, 46, 47).

18. Device according to one of the claims 1 to 17, characterised by use of shrinkable thread material for the formation of the sheathing (14, 46, 47) surrounding the prothesis (15, 45) in the compressed state on the probe or the catheter (11, 41) serving as such.

19. Device according to one of the claims 2 to 18, characterised thereby, that the sheathing (14, 46, 47), which can be drawn on, consists of several threads one extending parallelly to the other.

20. Device according to one of the claims 1 to 19, characterised thereby, that at least one further sheathing, which loosely surrounds the prothesis and permits a partial expansion of the prothesis during the drawing-on of the other sheathing as well as in its turn can be drawn on subsequently, is arranged between the prothesis (15, 45) and the sheathing (14, 46, 47) holding this in the radially compressed state.

21. Device according to one of the claims 1 to 19, characterised thereby, that the gaps between the stitches of the mesh (14, 46, 47), which surrounds the prothesis (15, 45) and holds it in the compressed state, are filled out and smoothed by gelatine or a similar substance which dissolves in the body of a patient.

22. Device according to one of the claims 6 to 21, characterised thereby, that at least one end of the prothesis (15, 45) in the compressed state is engaged over by a sleeve of the probe or the catheter and, in consequence of the axial shortening which arises on expansion, gets out of the retention provided by the sleeve.

23. Device according to one of the claims 5 to 22, characterised thereby, that that end of the prothesis (15, 45), which is remote from the entering end, bears against a radially protruding step or shoulder of the probe or against a sleeve (62) received on the latter.

24. Device according to one of the claims 6 to 23, characterised thereby, that on the use of a catheter (11, 41) as probe, the pull line (24, 52, 60) is introduced through a perforation, which penetrates the catheter wall in the proximity of one end of the prothesis, into the catheter passage, extends through the latter and reaches beyond the end of the catheter.

25. Device according to one of the claims 6 to 24, characterised by a twin-passage catheter as the probe receiving the prothesis (15, 45), wherein one passage serves for the advancing of the catheter by way of a guide wire and the pull line for the mesh surrounding the prothesis is led through the other passage.

26. Device according to one of the claims 1 to 25, characterised by the equipment of the thread material forming the pull line (24, 52, 60) and/or the sheathing (14, 46, 47) with a friction-reducing material.

27. Device according to one of the claims 1 to 26, characterised thereby, that at least the pull line (24, 52, 60) is formed as metal thread or equipped with an admixture of metal representable in an X-ray image.

28. Device according to one of the claims 1 to 27, characterised thereby, that the prothesis (15, 45), which is held in the radially compressed state by means of the sheathing (14, 46, 47), which can be drawn on, in the expanded state after removal of the sheathing enlarges in the manner of a trumpet towards its proximal end.

## Revendications

1. Dispositif de prothèse creuse implantable dans le corps d'un patient, en particulier dans un vaisseau sanguin ou autre cavité corporelle maintenu contre la force de rejet notamment dans les régions de section réduite et comprenant les moyens de maintien propre sur une section, caractérisé en ce que la prothèse (15) à auto maintien comporte une enveloppe extractible (14) constituée de fils, pouvant être fermée et de section réduite par pression et en ce que, à partir de l'enveloppe s'étend vers l'avant un ruban de tirage (24) fixé sur l'enveloppe et pouvant être tiré de l'arrière

2. Dispositif de prothèse creuse implantable dans le corps d'un patient, en particulier dans un vaisseau sanguin ou autre cavité corporelle, maintenu contre la force de rejet notamment dans les régions de section réduite et comprenant les moyens de maintien propre sur une section de sorte que la suppression de la pression provoque un auto maintien par dilatation de sa section, caractérisé en ce que la prothèse à auto maintien (45) est solidarisée avec une sonde (42), respectivement d'un cathéter (41) respectivement du cathéter 41 avec le fil courant à partir de l'extrémité distale (12) jusqu'à une extrémité d'introduction de la prothèse (45) constitué d'une première configuration de mailles (46) et au moyen d'un deuxième fil à partir de l'extrémité arrière de la sonde, une seconde configuration de mailles (47), , de sorte que sur la maille terminale (51) de la première configuration de maille (46), recouvre partiellement la deuxième configuration de mailles (47) en position de pression radiale et en ce que les deux configurations (46, 47) de mailles respectives, constituent des mailles terminales de forme cylindrique (51, 58) avec deux rubans de traction (52, 60) pouvant être tirés.

3. Dispositif selon la revendication 1 ou 2, caractérisé en ce que la prothèse (15, 45) est maintenue en pression radiale par l'enveloppe constituée d'une configuration de mailles pouvant être tirées et relâchées (14, 46, 47).

4. Dispositif selon la revendication 1, 2 ou 3, caractérisé en ce que les moyens pour tirer l'enveloppe (14, 46, 47, ) en position de pression radiale de maintien de la prothèse (15, 45) comprennent une sonde (11, 41) tel qu'un fil métallique de guidage et coulissent sur celui-ci.

5. Dispositif selon la revendication 1, 2 ou 3, caractérisé en ce que les moyens pour tirer l'enveloppe (14, 46, 47) en position de pression radiale de maintien de la prothèse (15, 45) sont fermement fixés sur l'extrémité d'introduction de la sonde (11,41).

6. Dispositif selon la revendication 5, caractérisé en ce que la prothèse (15, 45) est disposée sur l'extrémité d'introduction (12) de la sonde et autour d'un fil métallique de guidage coulissant dans le cathéter (11, 41).

7. Dispositif selon la revendication 5 ou 6, caractérisé en ce que la prothèse (15, 45) est emprisonnée sous une couche intermédiaire et une couche de base résistant au glissement de la sonde.

8. Dispositif selon l'une des revendications 1 à 6, caractérisé en ce que, la prothèse (15, 45) est entourée par des boucles ou analogues formant une configuration de mailles de métal ou de matériau fibreux synthétique constituant un revêtement cylindrique qui applique une pression radiale à l'encontre de la force élastique de rejet et en ce que après retrait de la force de pression, elle reste ancrée dans sa position.

9. Dispositif selon l'une des revendications 1 à 8, caractérisé en ce que la forme cylindrique de la prothèse (15, 45) est en position compressée par la configuration de mailles (14, 46, 47) de sorte que chaque boucle successive change de direction sur la prothèse et par traction sur les mailles alternées, le fil passe alternativement à gauche et à droite de la prothèse.

10. Dispositif selon la revendication 9, caractérisé en ce que la prothèse (15, 45) entourée par la configuration de mailles (14, 46, 47) est fermée par des crochets, noeuds ou analogues, la configuration de mailles constituant des boucles pouvant être tirées.

11. Dispositif selon les revendications 9 ou 10, caractérisé en ce que les noeuds pouvant être tirés alternativement autour de la prothèse (15, 45), des mailles de la configuration de mailles (14, 46, 47) se recouvrant respectivement l'une et l'autre ou sont disposés en rangées sensiblement axiales se recouvrant l'une et l'autre.

12. Dispositif selon une des revendication 1 à 11, caractérisé en ce que le ruban de tirage (24, 52) s'étend de l'extrémité d'introduction de la prothèse (15, 45) par des mailles et permet de tirer les mailles s'étendant sur la prothèse.

13. Dispositif selon l'une des revendications 1 à 12, caractérisé en ce que les extrémités de la configuration de mailles (14, 46, 47) constituées de matériau fibreux sont réunies au moyen de noeuds pouvant être tirés, tels que des noeuds glissants.

14. Dispositif selon l'une des revendications 3 à 13, caractérisé en ce que respectivement le début de la configuration de mailles (14, 45, 46) de matériau fibreux et une maille finale sur la sonde ou sur un cathéter (11, 41, 52) est fixé et au moyen des rubans de tirage (24, 52, 60) peut être tiré hors de leur fixation.

15. Dispositif selon la revendication 14, caractérisé en ce que le début (17, 48, 55) de la configuration de mailles (14, 46, 47) de matériau fibreux et lesdites mailles terminales (22, 51, 58) sont assemblées par pénétration dans des trous (18, 23, 23') de la sonde ou du cathéter.

16. Dispositif selon la revendication 15, caractérisé en ce que le début (48, 55) de la configuration de mailles (46, 47) de matériau fibreux est assemblé entre la sonde ou le cathéter et l'un desdits manchons élastiques (43, 54).

17. Dispositif selon l'une des revendications 14 à 16, caractérisé en ce que des noeuds peuvent être tirés de la configuration de mailles (14, 46, 47), une première maille sur la sonde ou le cathéter, consistant en une boucle de fixation (22, 51, 58) dont une extrémité dans la région desdits noeuds présente un ruban de tirage (24, 52, 60) et au moyen de ce dernier, les noeuds peuvent tirés.

18. Dispositif selon l'une des revendications 1 à 17, caractérisé par l'emploi pour maintenir la prothèse (15, 45) en position compressée sur la sonde ou un cathéter (11, 41) d'une enveloppe d'enrobage fermée (14, 46, 47).

19. Dispositif selon l'une des revendications 2 à 18, caractérisé en ce que l'enveloppe pouvant être tirée (14, 46, 47) est formée de plusieurs fils s'étendant parallèlement.

20. Dispositif selon l'une des revendications 1 à 19, caractérisé en ce que au moins une enveloppe supplémentaire est disposée entre la prothèse (15, 45) et l'enveloppe de pression radiale (14, 46, 47), l'enveloppe supplémentaire pouvant être tirée à l'intérieur de l'enveloppe extérieure.

21. Dispositif selon l'une des revendications 1 à 19, caractérisé en ce que les chambres intermédiaires entre les mailles entourant la prothèse (15, 45) sont revêtues d'une substance telle que de la gélatine ou autre matériau biocompatible.

22. Dispositif selon les revendications 6 à 21, caractérisé en ce qu'au moins une extrémité de la prothèse (15, 45) est maintenue comprimée par un manchon sur la sonde ou sur le cathéter de manière à constituer un étranglement axial dans lequel est maintenu le manchon.

23. Dispositif selon les revendications 5 à 22, caractérisé en ce que l'extrémité d'introduction de la prothèse (15, 45) présente un degré ou épaulement de la sonde sur laquelle s'appuie un manchon (62).

24. Dispositif selon l'une des revendications 6 à 23, caractérisé par l'utilisation d'un cathéter (11, 41) en tant que sonde et d'un ruban de tirage (24, 52, 60) passant à travers la paroi du cathéter au voisinage d'une extrémité de la prothèse conduit dans une lumière de cathéter de manière à pouvoir atteindre l'extrémité du cathéter.

25. Dispositif selon l'une des revendications 6 à 24, caractérisé en ce qu'on utilise comme prothèse (15, 45) un cathéter à double lumière jouant le rôle de sonde de sorte que la première lumière du cathéter reçoive un fil métallique et que l'autre lumière soit utilisée pour conduire un ruban de tirage.

26. Dispositif selon l'une des revendications 1 à 25, caractérisé en ce que la ligne de tirage (24, 52, 60) et/ou l'enveloppe (14, 46, 47) est recouverte d'un matériau fibreux avec un lubrifiant.

27. Dispositif selon l'une des revendications 1 à 26, caractérisé en ce que le ruban de tirage (24, 52, 60) est constitué au moins en partie d'un fil métallique ou recouvert d'un métal imperméable aux rayons X.

28. Dispositif selon une des revendications 1 à 27, caractérisé en ce que le moyen de tirage de l'enveloppe pouvant être tirée (14, 46, 47) est maintenue en pression radiale avec la prothèse (15, 45) à distance de l'enveloppe, son extrémité arrière s'évasant en forme en trompette.
